# EUROPEAN PATENT APPLICATION

(11) **EP 2 201 948 A1**
(43) Date of publication of application: **30.06.2010**
(21) Application number: 08172954.3
(22) Date of filing: 26.12.2008
(51) Int. Cl.: A61K 31/19, A61K 31/7004, A61K 33/06, A61K 33/14, A61P 3/12

(54) **Maintenance fluid for animals**

(71) Applicant: Laboratorium Dr. G. Bichsel AG, 3800n Interlaken (CH)
(72) Inventor: Gerber, Vinzenz, CH-3012 Bern (CH); Bichsel, Tobias, ch-3812 (CH)
(74) Representative: Grosfillier, Philippe

(57) **Abstract**

Maintenance fluid for fluid therapy in animals comprising the following components/quantities:
- Na+ : 38 - 50 mmol/l
- K+ : 20 - 40 mmol/l
- Ca++ : 5 - 11 mmol/l
- Cl- : 50 - 70 mmol/l
- Mg++ : 5 - 12 mmol/l
- Glu-Anhydrid : 60 - 100 mmol/l

## Description

### Field of invention

The present invention relates to fluid therapy in animals such as horses.

### State of the art

Fluid administration for maintenance purposes is commonly used in veterinary hospitals, particularly in equine hospitals.

As discussed in the Lyon Lee article ("Fluid & Electrolyte Therapy", Veterinary Health Sciences, Veterinary Surgery I, VMED 7412), animal maintenance fluids are hypotonic crystalloids that are low in sodium, chloride but high in potassium compared to normal plasma compositions. They may or may not contain dextrose.

State-of-the-art maintenance fluids generally comprise at least sodium and chloride. They preferably also comprise potassium and may also include calcium, magnesium and lactate.

In human medicine different maintenance and nutrition fluids are available. The most common maintenance and nutrition fluids are Plasma-Lyte A^{™}, GIucoSaline^{™} and Nutriflex^{™}.

Examples of maintenance and nutrition fluids for humans are listed in Tables 1 and 2.

**Table 1 : Maintenance fluids in human medicine**

| | Plasma-Lyte A^{™} | GIucoSaline^{™} 1:1 | GlucoSaline^{™} 2:1 | GIucoSaline^{™} 4:1 | |
|---|---|---|---|---|---|
| Na+ | 140.1 | 77 | 51 | 31 | mmol/l |
| K+ | 4.96 | - | - | - | mmol/l |
| Ca++ | - | - | - | - | mmol/l |
| Mg++ | 1.48 | - | - | - | mmol/l |
| CI- | 97.93 | 77 | 51 | 31 | mmol/l |
| Glucose | - | 25 | 33.3 | 40 | g/l |
| Glukonat | 23.01 | - | - | - | mmol/l |
| Acetat | 27.05 | - | - | - | mmol/l |
| Osmolarity | 295 | 293 | 288 | 284 | mosmol/l |

**Table 2 : Nutrition fluids in human medicine**

| | Nutriflex^{™} special 70/240 | Nutriflex^{™} plus 48/150 | Nutriflex^{™} basal 32/125 | Nutriflex^{™} peri 40/80 | |
|---|---|---|---|---|---|
| Amino acids | 70 | 48 | 32 | 40 | g/l |
| Glucose | 240 | 150 | 125 | 80 | g/l |
| Na+ | 40.5 | 37.2 | 49.9 | 27 | mmol/l |
| K+ | 25.7 | 25 | 30 | 15 | mmol/l |
| Ca++ | 4.1 | 3.6 | 3.6 | 2.5 | mmol/l |
| Mg++ | 5 | 5.7 | 5.7 | 4 | mmol/l |
| Phosphate | 14.7 | 20 | 12.8 | 5.7 | mmol/l |
| CI- | 49.5 | 35.5 | 50 | 31.6 | mmol/l |
| Acetat | 22 | 22.9 | 35 | 19.5 | mmol/l |

There are different reasons why these maintenance fluids are not used in equine medicine.

Plasma-Lyte A^{™} is an infusion solution for humans, but commonly used in small animal medicine and not adapted for horses. The sodium and chloride concentrations are with 140.1 and 97.93 mmol/l, respectively, to high. On the other hand the concentrations of potassium and magnesium are not adequate for hospitalized horses. The potassium and magnesium concentrations in MS1 are well balanced and much higher than those in Plasma-Lyte A^{™}. A great application constriction is that Plasma-Lyte A^{™} does not provide any calcium and glucose, which are very important for horses, especially for those with gastrointestinal problems.

GIucoSaline^{™} in different concentrations are often used in human hospitals. This infusion solution only offers sodium, chloride and glucose to the patient, therefore it is not adequate for horses. There would be a risk for hypernatremia, hyperchloremia and additionally a lack of magnesium, calcium and potassium.

Nutriflex^{™} is also available in different compositions. In general, the ingredients are appropriate for hospitalized horses, but the concentrations are not adapted. In all four different infusions the glucose concentrations are much too high for horses and would lead to glucosuria. Potassium and sodium concentrations would be appropriate for these horses, but the solutions do not contain enough calcium, magnesium and chloride.
Apart from the fact that all the human maintenance and nutrition fluids are too expensive and only available in small bags, none of these infusions covers the demands of horses and particularly not of patients with gastrointestinal problems.

Furthermore the available bags are too small, they contain between 1 to 2 l and horses often need more than 20 l of infusions. It would be too expensive and also too conspicuous, because the infusion bag only contains one liter and therefore has to be changed very frequently. The maintenance infusion rate is about 1.5 ml/kg/h, for a 500kg horse one liter of infusion would be administered in 75 min. One of the most commonly used maintenance fluids in horses is Normosol M ™ which contains the following components :

| | | |
|---|---|---|
| - | Na | : 40 mmol/l |
| - | K | : 13 mmol/l |
| - | Cl | : 40 mmol/l |
| - | Mg | : 1.5 mmol/l |
| - | Acetate | : 16 mmol/l |

This fluid is however not adapted to the animal requirements, especially when the treatment should be made during several days.

In summary, no maintenance fluids exist, which are specifically developed and adapted to the needs of animals in particular of horses.

There is therefore a need to improve existing maintenance fluids in order to better meet the animal requirements, in particular of K, Mg, Ca and glucose. Those requirements are particularly important when fluid therapy has to be continued over several days.

### General description of the invention

The present invention relates to an improved maintenance fluid with respect to state-of-the-art maintenance fluid.

In an unexpected manner the inventors have observed that such an improved maintenance fluid can be obtained if it contains the following components and related quantities :

| | |
|---|---|
| - Na+ : | 38 - 50 mmol/l |
| - K+ : | 20 - 40 mmol/l |
| - Ca++ : | 5 - 11 mmol/l |
| - Cl- : | 50 - 70 mmol/l |
| - Mg++ : | 5 - 12 mmol/l |
| - Glu-Anhydrid : | 60 - 100 mmol/l |

In one embodiment the maintenance fluid according to the invention also comprises lactate in an amount of 30 to 50 mmol/l.

Preferably the maintenance fluid comprises the following components/quantities :

| | |
|---|---|
| - Na+ : | 40 - 45 mmol/l |
| - K+ : | 25 - 35 mmol/l |
| - Ca++ : | 6 - 10 mmol/l |
| - Cl- : | 55 - 65 mmol/l |
| - Mg++ : | 6 - 10 mmol/l |
| - Glu-Anhydrid : | 70 - 90 mmol/l |
| - Lactate: | 35 - 45 mmol/l |

One of the most preferred embodiments of the invention concerns a maintenance fluid comprising the following components and precise related quantities :

| | |
|---|---|
| - Na+ : | 42 mmol/l |
| - K+ : | 30 mmol/l |
| - Ca++ : | 8 mmol/l |
| - Cl- : | 62 mmol/l |
| - Mg++ : | 8 mmol/l |
| - Glu-Anhydrid : | 83 mmol/l |
| - Lactate : | 42 mmol/l |

The present invention is particularly efficient in horse fluid therapy, especially for horses having gastrointestinal problems.

### Detailed description of the invention

The invention will be better understood below with the use of an example in which one maintenance fluid (MS1) according to the invention is compared with a state-of-the-art maintenance fluid (Normosol M^{™}).

**Table 1: Composition of Normosol M^{™ and} MS1**

| | **Normosol M^{™}** | **MS1** | |
|---|---|---|---|
| Na + | 40 | 42 | mmol/l |
| K+ | 13 | 30 | mmol/l |
| Ca++ | - | 8 | mmol/l |
| Cl- | 40 | 62 | mmol/l |
| Mg++ | 1.5 | 8 | mmol/l |
| Acetat | 16 | - | mmol/l |
| Lactate | - | 42 | mmol/l |
| Glukose | - | 15 | g/l |
| Osmolarity | 111 | 275 | mosmol/l |

Normosl M^{™} was tested on healthy, fasted horses during a 5 day infusion period. MS1 was tested on horses with gastrointestinal problems at the equine horse clinic in Berne and Zurich. Horses of both studies did not receive any food or water.

Horses need enough potassium especially when they do not eat because then it decreases markedly (Groenendyl et al. 1988; Tasker 1967). Also diuresis induced by excess sodium will exacerbate potassium losses, since kidneys of horses are poorly able to conserve potassium (Michell 1983; Rumbaugh et al. 1982; Rose 1990; Johnson 1998; Schott 2003; Tasker 1967). Potassium loss can be further exacerbated in horses with diarrhea (Carlson 1979). For MS1 a large amount of potassium was added in the solution (30 mmol/l) compared to Normosol M^{™}, which contains much less potassium (13 mmol/l). Surprisingly, this concentration was adequate and resulted in a stable potassium blood concentration of about 3.36 - 3.7 mmol/l (normal values: 2.8 - 4.5 mmol/l).

Horses that are off-feed commonly show mild decreases in calcium and magnesium concentrations. This is even more pronounced in equine patients with gastrointestinal diseases (Johansson et al. 2003; Lopes et al. 2004; Navarro et al. 2005; Seahorn and Cornick-Seahorn 1994; Toribo et al. 2001).

Decreased intestinal absorption, increased renal excretion and shifts from the extracellular to the intracellular space can lead to hypomagnesemia and hypocalcemia (Mogg 2001). Hospitalized horses with colic often show hypomagnesemia. Based on these findings it is very important for horses with gastrointestinal problems to receive an infusion solution which contains enough magnesium and calcium. As mentioned above, Normosol M^{™} is a commonly used maintenance fluid, but one of its relevant disadvantages is that it contains relatively small amounts of magnesium and no calcium. By contrast, the advantage of MS1 is, that it contains 8 mmol/l of calcium and also 8 mmol/l of magnesium. The calcium blood concentrations of Normosol M^{™} were about 1.5 mmol/l on the average and the blood concentrations of MS1 were about 2.5 - 2.66 mmol/l.

Amazingly, the blood calcium concentrations of horses having received MS1 were within the normal range (2.5 - 3.45 mmol/l) and not beyond, even though the calcium concentration of MS1 was much higher than the concentration of Normosol M^{™}. The same also applies for the magnesium concentration. There the concentration in MS1 was 5.3-fold increased compared to Normosol M^{™}, but the blood concentrations were both in the normal range.

Hospitalized horses also have an increased need of chloride, especially horses with reflux, diarrhea and ileus, because they have increased losses. An increased chloride concentration of about 62 mmol/l was chosen for MS1 with the intention to cover the increased chloride demand of horses with reflux, diarrhea and ileus. Finally the chloride concentrations of the blood were about 97.53 - 100.2 mmol/l, which is within the normal range of blood chloride concentrations (95 - 105 mmol/l).

In addition to replacing on-going water and electrolyte losses, hospitalized horses with decreased or absent feed intake would benefit from an intravenous fluid containing calories (glucose). Energy requirements may further be increased in sick animals compared to healthy individuals because of the increased metabolism and healthy process. The fact that MS1 contains glucose is a big advantage, compared to Normosol M^{™}, which does not contain any glucose. To MS1 15 g/l of glucose has been added. A certain risk for hyperglycemia, hyperinsulinemia, volume overload, and diuresis as a result, was taken into account. Glucosuria was observed at the beginning of infusion. During the further course of the maintenance period, however, glucosuria markedly decreased and disappeared indicating that the patient adapted to and then tolerated the glucose administered.

In conclusion, MS1, and more generally the maintenance fluid according to the invention, is superior to Normosol M^{™} and other existing maintenance fluids, especially for fasting horses with gastrointestinal problems.

## Claims

1. Maintenance fluid for fluid therapy in animals comprising the following components/quantities:
| | |
|---|---|
| - Na+ : | 38 - 50 mmol/l |
| - K+ : | 20 - 40 mmol/l |
| - Ca++ : | 5 - 11 mmol/l |
| - Cl- : | 50 - 70 mmol/l |
| - Mg++ : | 5 - 12 mmol/l |
| - Glu-Anhydrid : | 60 - 100 mmol/l |

2. Maintenance fluid according to claim 1 furthermore comprising :
| | |
|---|---|
| - Lactate : | 30 - 50 mmol/l |

3. Maintenance fluid according to claim 2 comprising the following components/quantities :
| | |
|---|---|
| - Na+ : | 40 - 45 mmol/l |
| - K+ : | 25 - 35 mmol/l |
| - Ca++ : | 6 - 10 mmol/l |
| - Cl- : | 55 - 65 mmol/l |
| - Mg++ : | 6 - 10 mmol/l |
| - Glu-Anhydrid : | 70 - 90 mmol/l |
| - Lactate: | 35 - 45 mmol/l |

4. Maintenance fluid according to claim 3 comprising the following components/quantities :
| | |
|---|---|
| - Na+ : | 42 mmol/l |
| - K+ : | 30 mmol/l |
| - Ca++ : | 8 mmol/l |
| - Cl- : | 62 mmol/l |
| - Mg++ : | 8 mmol/l |
| - Glu-Anhydrid : | 83 mmol/l |
| - Lactate: | 42 mmol/l |

5. Maintenance fluid according to one of the previous claims for fluid therapy in horses.

6. Maintenance fluid according to claim 5 for fluid therapy in horses with gastrointestinal problems.
